# EUROPEAN PATENT APPLICATION

(11) **EP 4 215 181 A1**
(43) Date of publication of application: **26.07.2023**
(21) Application number: 22152795.5
(22) Date of filing: 21.01.2022
(51) Int. Cl.: A61K 9/00, A61K 47/32, A61K 31/565, A61K 31/57, A61P 15/18

(54) **AN INTRAVAGINAL RING**

(71) Applicant: Sever Pharma Solutions, 212 15 Malmö (SE)
(72) Inventor: DE GRAAFF, Wouter, 212 15 Malmö (SE); VAN LAARHOVEN, Hans, 212 15 Malmö (SE)
(74) Representative: Holme Patent A/S

(57) **Abstract**

The present invention relates to an intravaginal ring (1) a core (2) comprising a first ethylene-vinyl acetate copolymer (3) having a vinyl acetate content from 28 to 40%, and at least 10 wt% of an estrogenic steroid (4) based on the weight of the core, and a sheath (5) comprising a second ethylene-vinyl acetate copolymer (6) having a vinyl acetate content from 18 to 28%, and at least 10 wt% of a progestational steroid (7) based on the weight of the sheath. Using the intravaginal ring according to the present the inventors have found that it is possible to attain independent and optimal release of the two active ingredients; an estrogenic steroid and a progestational steroid, without the need for complex assembly of parts and without the need to use sophisticated multi-layer extrusion technology.

## Description

The present invention relates to an intravaginal ring, a method of manufacturing said ring and the use of said ring.

Various types of intravaginal rings (IVRs) have been developed for the controlled and sustained release of active ingredients preferably by diffusion through the surface of the ring.

Examples of such devices are the Estring^{®}, Femring^{®}, and Nuvaring^{®}, all of which provide controlled and sustained release of steroid molecules over a prolonged period, e.g. several weeks/months. In addition to preventing undesired pregnancies, the device provides several advantages: its use is controlled by the female; it allows for a better regulated dose of drug without attention by the user; and it avoids the destruction (by the intestine and by first pass through the liver) of an appreciable portion of the daily dosage of the drugs compared to their orally delivered counterparts.

These known vaginal rings have been found particularly useful for the release of steroids, whose relatively small molecular size and substantially water-insoluble nature permit effective permeation through the hydrophobic elastomer/polymer, such that therapeutic concentrations may be readily achieved in the body.

However, diffusion in polymers is complex and is known to depend on a number of different factors, e.g. temperature, the manufacturing process, the solubility and diffusivity of the drug in the polymer, the surface area of the drug reservoir, the distance the drug must diffuse through the device to reach its surface and the molecular weight of the drug. Consequently, it remains a challenge to understand, predict and control the diffusion of small and large molecules in polymer systems. In this respect, the use of intravaginal rings to deliver drugs requires a design that regulates the release rate so as to reliably provide the user with the appropriate daily dose throughout the lifetime of the device.

In reservoir systems, i.e. a drug loaded core surrounded by a non-medicated membrane/sheath, the drug first partitions into the sheath from the reservoir and then diffuses to the other side of the sheath, where it is taken up by the receiving medium. While the reservoir is saturated, a constant concentration gradient of drug is maintained in the membrane, the rate of drug flux is constant, and zero order release is achieved. However, when drug concentration in the reservoir falls, the gradient across the membrane and the release rate of the drug also decreases.

Furthermore, reservoir systems can be difficult to fabricate reliably, and pinhole defects and cracks in the sheath surrounding the reservoir, can lead to dose dumping, i.e. unintended, rapid drug release over a short period of time.

Simultaneous drug delivery/release finds application in a number of different areas. However, the placement of a blend of drugs in a single intravaginal ring in a proportion equal to the desired delivery rate ratio will almost never achieve the desired result. In many cases, the drugs will not diffuse together through the surface or membrane at the same ratio, as they exist in the blend. The ratio would instead be dependent on the inherent ratio of the normalized permeation rates for the drugs through e.g. a rate-controlling membrane. Flexibility would therefore be limited to the selection of suitable polymer candidates for the sheath. Accordingly, the range of, and degree of control over, the delivery rate ratio, is extremely limited.

Of course the need for maintaining a specified delivery rate ratio can be met by using a separate intravaginal ring for each drug. However, this is clearly undesirable, since the presence of two or more intravaginal rings will compound the disruption which even a single ring might create in the normal physiological activity of an animal or human. In addition, if one intravaginal ring malfunctions, the desired delivery ratio will be lost. Further, complete therapy in a single intravaginal ring is more acceptable to patients and more efficient to insert and remove.

Adjustment of a specified delivery rate can also be met by two or multi-compartments intravaginal rings, and ring bodies containing drug release capsules. The industrial scale manufacturing of such rings are however complex and expensive.

A further problem with the known intravaginal rings arranged for releasing more than one drug is that such rings usually show sub-optimum release patterns for the different drugs, whereas it is generally preferred that all drugs are released in a controlled rate during a specified duration of time.

Thus, there is a demand for a novel intravaginal ring arranged for releasing two active ingredients/drugs in a controlled manner and in the correct ratio, and a method for manufacturing the system that is simple and inexpensive.

Thus, it is a first aspect of the present invention to provide an intravaginal ring, which can be loaded with both an estrogenic steroid and a progestational steroid and wherein each steroid is released at a controlled rate independently of the other steroid.

In a second aspect according to the present invention is provided an intravaginal ring that reduces the variability of the release rate of steroids over time.

In a third aspect according to the present invention a provided an intravaginal ring in which the known problems relating to complicated and expensive manufacturing processes, dose dumping and initial drug burst are eliminated, and which at the same time provides a substantially zero-order release rate of the estrogenic steroid.

In a fourth aspect according to the present invention a provided an intravaginal ring which is stable at room temperature.

The novel and unique features whereby these and further aspects are achieved according to the present invention is by providing intravaginal ring comprising
- a core comprising a first ethylene-vinyl acetate copolymer having a vinyl acetate content from 26 to 40%, and an estrogenic steroid, and
- a sheath comprising a second ethylene-vinyl acetate copolymer having a vinyl acetate content from 20 to 40%, and at least 10 wt% of an progestational steroid based on the weight of the sheath.

The intravaginal ring according to the invention relates to a system that comprises a core surrounded, at least partly but preferably completely, by a sheath. However contrary to the conventional systems of this kind, the sheath of the present invention also comprises a high concentration of an active ingredient, whereby simultaneous release of two steroids is provided.

Dual administration of two steroids finds application in a number of different areas, e.g. in contraceptive rings and in rings providing hormone replacement.

For such intravaginal rings it is required to release the two steroids simultaneously and at the same time. It is therefore necessary to adjust the release rate of these steroids independently to the physiological optimal rate (mg/day). Using the intravaginal ring according to the present invention the inventors have found that it is possible to attain independent and optimal release of the two steroids; an estrogenic steroid and a progestational steroid without the need for complex assembly of parts and without the need to use sophisticated multi-layer extrusion technology.

Using an intravaginal ring in which the concentration of the progestational steroid in the sheath is at least 10wt%, it is ensured that the desired near zero-order release behavior of the estrogenic steroid in the core are observed for a longer period of time. In addition such high concentrations of the active ingredient are associated with good physical stability of the progestational steroid.

By substantially zero order is meant that a substantially constant amount of the estrogenic steroid is released over a given period of time. In some embodiments, the system exhibit a substantially zero order release profile of the estrogenic steroid over a treatment period of at least 14 days, preferably at least 28 days, and even more preferred around two to three months.

As discussed earlier it is a well known problem that the release rate of the active ingredient in the core decreases as active ingredient(s) that is deeper inside the core/reservoir must diffuse to the surface. However loading the sheath with a high concentration of a progestational steroid, the inventors of the present invention has found that the release rate of the estrogenic steroid in the core slightly increases over time, thereby compensating for the further distance said steroid must travel to the surface of the intravaginal ring for being released to the surroundings, thereby providing a substantially zero-order relate rate of the estrogenic steroid during the desired treatment period.

It is important that the progestational steroid is dispersed and/or incorporated in the second ethylene-vinyl acetate copolymer to an extent sufficient to control the diffusion rate of the estrogenic steroid through the sheath.

Without being bound by theory, it is believed that the progestational steroid in the sheath will function as a filler and control the release rate of the estrogenic steroid. When the progestational steroid present in the sheath is released to the surroundings, the concentration of said progestational steroid is reduced and it is believed that that diffusion of water into the sheath may be facilitated leaving behind an empty porous matrix and/or empty pockets/holes and/or the sheath may collapse thereby ensuring the desired zero-order release profile of the estrogenic steroid. Thus, it is believed that the space initially occupied by the progestational steroid leaves behind an empty porous matrix which may become water filled due to ingress of water and/or may leave behind empty pockets/holes. This is contrary to the conventional findings in which the release rate of an active ingredient in a core surrounded by a non-medicated sheath slightly decreases over time, i.e. the desired zero order release rate cannot be maintained over a desired treatment period for such conventional vaginal rings.

In some embodiments it is preferred that at least 15 wt% of the progestational steroid is dispersed in the second ethylene-vinyl acetate copolymer, even more preferred at least 20 wt%, and even more preferred at least 25 wt%.

In order to ensure that the estrogenic steroid can be released through the sheath it is preferred that the sheath comprises a concentration of the progestational steroid based on the weight of the sheath of not more than 40 wt%, preferably 35 wt% or below.

The presence of the progestational steroid in the relatively high concentrations in the sheath will not only lead to an increase in the mean path length the molecules of the estrogenic steroid have to travel between two points in the sheath, but will also reduce the amount of the estrogenic steroid which can be dissolved in the second ethylene-vinyl acetate copolymer of the sheath, accordingly decreasing the release rate of the estrogenic steroid though said sheath. This will provide a reliable release rate and a lower initial burst of the estrogenic steroid. Accordingly, the sheath can be made smaller, providing a smaller product with a significant lower burst of the estrogenic steroid.

It is in this respect preferred that the progestational steroid is incorporated and/or dispersed in the second ethylene-vinyl acetate copolymer in the form of particles, preferably crystals. Such particles/crystals will form a repository of solid, undissolved crystals which will act as seed crystals i.e. as a slow release depot. Over time, when the progestational steroid is delivered to the surroundings, some of the crystals will be unlocked in the second ethylene-vinyl acetate copolymer, thereby providing a prolonged release of the progestational steroid. Furthermore, the stability of the progestational steroid in the intravaginal ring is improved when the progestational steroid is incorporated into the sheath as undissolved particles/crystals.

Without being bound by theory it is believed that maintaining a concentration of the progestational steroid at high concentrations in the sheath it is assumed that a porous network path is created by crystals and wherein a number of sites/openings/pores in the matrix of the second ethylene-vinyl acetate copolymer remains empty, ensuring that the estrogenic steroid only can be released through a tortuous path in the sheath, thereby the diffusion length increases and the release rate is controlled.

In order to obtained the desired zero order release profile of the estrogenic steroid during the treatment period, it is preferred that the estrogenic steroid is dissolved in the first ethylene-vinyl acetate copolymer and in these embodiments the estrogenic steroid is preferably present in the first ethylene-vinyl acetate copolymer at concentration at or below the saturation concentration of said estrogenic steroid at 25°C.

Since it is expected that some of the estrogenic steroid will redistribute throughout the intravaginal ring upon storage, i.e. the concentration of steroid in the core will drop as a part of the steroid will diffuse into the sheath, the term "below the saturation concentration" refers to the concentration of the estrogenic steroid in the core measured in an equilibrated intravaginal ring, i.e. when an equilibrium of the estrogenic steroid has been reached in the intravaginal ring.

Alternatively, the estrogenic steroid may be present in the core in the form of particles, preferably crystals, for the same reasons as disclosed for the progestational steroid. In this way the stability of both the estrogenic steroid and progestational steroid, and accordingly the intravaginal ring according to the intention, is improved.

When the estrogenic steroid is present as particles in the core, it is preferred that said estrogenic steroid is dispersed and/or incorporated in the first ethylene-vinyl acetate copolymer of the core in a concentration of at least 10 wt% of the total weight of the core, preferably at least 15 wt%, and even more preferred at least 20 wt%.

It must be noted that even though it is known to have small concentrations of active ingredient in a sheath surrounding a drug loaded core, see e.g. WO2013/120888, it is not known to have a progestational steroid in the sheath in the high concentrations claimed in the present invention. Small concentrations of active ingredient i.e. concentrations well below 10 wt%, will have no or only a very limited effect on the release rate of the active ingredient in the core, and are accordingly not relevant for the present invention.

A person skilled in the art will based on the context of the present invention understand that it will be possible to control and/or adjust the diffusion rate through the sheath by varying the amount/concentration of the progestational steroid in said sheath, by using different grades of the second ethylene-vinyl acetate copolymer, and/or by using different particle sizes of the progestational steroid or blends of different particles sizes.

It is however preferred that the particles/crystals of the progestational steroid and optionally the estrogenic steroid have a mean particle size of between 3 µm and 40 µm, preferably between 8 µm and 24 µm, and even more preferred between 10 and 24 µm as it has been proven that such particle sizes provide the desired near zero order relate rate for a prolongs period of time, i.e. for at desired treatment period of at least 14 days, preferably at least 28 days.

As used herein, the term "crystals" refers to particles of the active ingredient arranged in a ordered microscopic structure, forming a crystal lattice. The term "crystal size" or "particle size" refers to a crystal's or particle's mean particle diameter. Particle size and particle size distribution can be measured using, for example, a Malvern laser scattering particle size analyzer, or any other particle size measurement apparatus or technique known to person's skill in the art. As used herein, the term "crystal size" or "particle size" relates to the particle distribution diameter of the particle. As an example can be mentioned that D90 means that 90% of the particles have a diameter below the given value when measured using laser diffraction.

The first and second ethylene-vinyl acetate copolymers used in the intravaginal ring of the present invention are suitable for placement in the vaginal tract, i.e. the materials are nontoxic and non-absorbable in a patient or an animal, and are considered to have both excellent mechanical and physical properties.

The vinyl acetate concentration of the EVA-copolymer determines the rate of diffusion of the active ingredient through the system and generally, the lower the vinyl acetate concentration, the slower the active ingredient will be release from the copolymer or migrate through it.

The second ethylene-vinyl acetate copolymer has a vinyl acetate content from 20 to 40%, such as e.g. 24%, 28%, 33% or 40% as these materials will provide the desired release profile through the sheath, e.g. by ensuring that the progestational steroid release rate is at the optimal level.

In order to provide the desired zero order release profile of the estrogenic steroid it is preferred that the core comprises an ethylene-vinyl acetate copolymer with a vinyl acetate content from 26 to 40%, preferably 26%, 33% or 40%.

In one embodiment of the present invention the first and second ethylene-vinyl acetate copolymer have the same vinyl acetate content, i.e. the first and second ethylene-vinyl acetate copolymer are the same.

When a specific vinyl acetate content e.g. 20% is mentioned it refers to the content provided by the manufacture. However, manufactures may use different internal analytical methods for determining vinyl acetate content, and there may therefore be variations in the range of 1 - 2 % in the actual vinyl acetate content depending on the manufacture. Thus, in the present invention the vinyl acetate content refers of the vinyl acetate content in the ethylene-vinyl acetate copolymer determined by high resolution NMR according to standard methods.

The intravaginal ring according to the invention is a dual-drug delivery ring i.e. it comprises both an estrogenic steroid and a progestational steroid. In a preferred embodiment the estrogenic steroid is estradiol and the progestational steroid is selected from the group consisting of a progestogen, etonogestrel, levonorgestrel, d-1-norestrel and norethindrone, preferably levonorgestrel. The steroids can be selected for preventing contraception, or to treat a conditions, e.g. vaginal atrophy, or for hormone replacement therapy e.g. relating to symptoms associated with menopause, such as hot flashes.

The intravaginal ring according to the invention is adapted to deliver pharmaceutically effective amounts of the steroids. By "pharmaceutically effective," it is meant an amount, which is sufficient to affect the desired physiological or pharmacological change in the subject. This amount will vary depending upon such factors as the potency of the steroid, the desired physiological or pharmacological effect, and the time span of the intended treatment. Those skilled in the arts will be able to determine the pharmaceutically effective amount for any given steroid in accordance with standard procedures.

The thickness of the sheath, which is the outer layer of the intravaginal ring according to the present invention, can be varied to further control the release rate of the estrogenic steroid and the progestational steroid.

In one embodiment the thickness of the sheath is between 0.05 mm and 3 mm. Said thickness can preferably be between 0.05 mm and 2 mm, more preferably between 0.1 mm and 2 mm and even more preferably between 0.2 mm and 0.6 mm. In certain embodiments the thickness of the sheath is 100 µm, 200 µm, 300 µm, 400 µm, 500 µm, or 600 µm.

A person skilled in the art will in view of the present invention understand that a thin sheath can contain less active ingredient than a thicker sheath; and that the concentration of the progestational steroid in the sheath should be enough to sustain release over the desired treatment period. By using an ethylene-vinyl acetate copolymer grade with higher or lower vinyl acetate-content the sheath's thickness can be altered while maintaining essentially the same average release rate of the estrogenic steroid. For instance, if a thicker skin is desired because more of the progestational steroid has to be accommodated in the sheath, an ethylene-vinyl acetate copolymer grade with higher vinyl acetate content can be chosen.

The core preferably has a round cross-section with a cross-sectional diameter of between 2 and 8 mm, more preferably between 3 mm and 6 mm and even more preferably around 4 mm.

The dimensions of the intravaginal ring may vary depending upon the anatomy of the subject, the amount of active ingredient to be delivered to the patient, the time over which the active ingredient is to be delivered, the diffusion characteristics of the active ingredient and other manufacturing considerations. The only requirement being that the intravaginal ring should be flexible enough to enable bending and insertion inside the vaginal cavity and rigid enough to withstand the expulsive forces of the vaginal musculature without causing abrasion to the vaginal epithelium. The outer diameter of such intravaginal rings may range, e.g., from about 45 mm to about 65 mm, and/or the length of the fiber elements forming the intravaginal ring may have a length from 150 to 170 mm, preferably from 154 to 160 mm, such as about 157 mm.

In the context of the present invention the term intravaginal ring, also contemplates ring designs or structures, which have other shapes, e.g. polygonal shapes and/or wavy shapes, or where the structure is not a complete and/or closed circle/shape.

In a preferred embodiment the intravaginal ring comprises
- a core comprising a first ethylene-vinyl acetate copolymer having a vinyl acetate content of 28% or 33%, and a concentration of an estrogenic steroid below the saturation concentration at 25°C, and
- a sheath comprising a second ethylene-vinyl acetate copolymer having a vinyl acetate content of 24%, 28%, 33% or 40%, and 30 wt% of an progestational steroid based on the weight of the sheath.

The core of said preferred embodiment has a cross-sectional diameter of 4 mm and the sheath a thickness of 600 µm. The diameter of the ring is 54 mm.

In a preferred embodiment the average release rate of estradiol is from 80 µg/day to 160 µg/day during a treatment period of 28 days, and preferably a near zero order release rate is provided.

In additional or alternatively, the average release rate of progestogen is from 5 mg/day to 10 mg/day for a treatment period of 28 days. It is expected that the release rate of progestogen will decrease during the treatment period as the content of progestogen in the sheath is depleted.

People skilled in the art will based on the present application understand that combinations of the average release rates of estradiol and estradiol is contemplated within the scope of the present inventing. For instance, in one intravaginal ring the average release rate of estradiol is 80 µg/day and the average release rate of progestogen is 10 mg/day, in a different embodiment the average release rate of estradiol is 100 µg/day and the average release rate of progestogen is 5 mg/day, in a third embodiment the average release rate of estradiol is 160 µg/day and the average release rate of progestogen is 8 mg/day, etc.

In a preferred embodiment according to the present invention the intravaginal ring does not comprise more than the two steroids, i.e. an estrogenic steroid and a progestational steroid, and/or does not comprise further cores and/or layers such as sheaths and membranes, i.e. the intravaginal ring according to the invention consists of a single core and a single sheath completely surrounding said core, and wherein the estrogenic steroid in the core and the a progestational steroid in the sheath.

The present invention also relates to a method of manufacturing the intravaginal ring according to the present invention.

Said method comprises
a. providing a core comprising a first ethylene-vinyl acetate copolymer having a vinyl acetate content from 26 to 40%, and an estrogenic steroid,
b. providing a sheath of comprising a second ethylene-vinyl acetate copolymer having a vinyl acetate content from 20 to 40%, and at least 10 wt% of an progestational steroid based on the weight of the sheath, and
c. co-extruding the core and sheath into a fiber, and
d. cutting the fiber into an appropriate length thereby providing a fiber element, and
e. combining the ends of fiber element to form the intravaginal ring.

As the core and sheath are co-extruded a very simple and inexpensive embodiment according to the invention is provided, however the cores and sheath can be formed in separate injection molding or extrusion steps if preferred. Injection molding and extrusion are well known in the art and will not be discussed further in this application.

It is preferred that the method further comprises a cooling step in which the provided fiber or fiber element is cooled to a temperature of 20°C or below for providing the crystals of the steroid in the sheath and optionally the core. This may e.g. be obtained by placing the fiber into a cooling water bath.

Without being bound by theory, the inventors believe that the crystals formed in the sheath and optionally the core is caused by the kinetics of re-crystallization. The relatively high concentrations of active ingredient in the sheath and optionally the core, will result in a higher concentration of "seed" crystals, upon which recrystallization can occur when the fiber is cooled after co-extrusion.

It is preferred that said cooling step is performed immediately after step c., i.e. as fast as is practically possible from a production point of view, i.e. preferably within less than 30 minutes from completion of the fiber in step c.

It is preferred that the rings are produced by heat welding the fiber ends together without the addition of further EVA material or adhesive.

The invention will be explained in greater detail below, describing an exemplary embodiment of the intravaginal ring according to the invention
Fig. 1 shows a perspective view of a preferred embodiment of a vaginal ring according to the invention,
Fig. 2 shows the simulated average release of progesterone for ring AE3,
Fig. 3 shows the simulated average release of estradiol for ring AE3,
Fig. 4 shows the simulated progesterone release and the impact of sheath thickness on early depletion,
Fig. 5 shows the influence of cross-sectional diameter on the release profile of estradiol,
Fig. 6 shows the simulated average release of estradiol for ring BF1,
Fig. 7 shows the simulated average release of progesterone for ring BF1,
Fig. 8 shows the simulated estradiol release and the impact of sheath thickness on release rate (rings BF1, BF2 and BF3), and

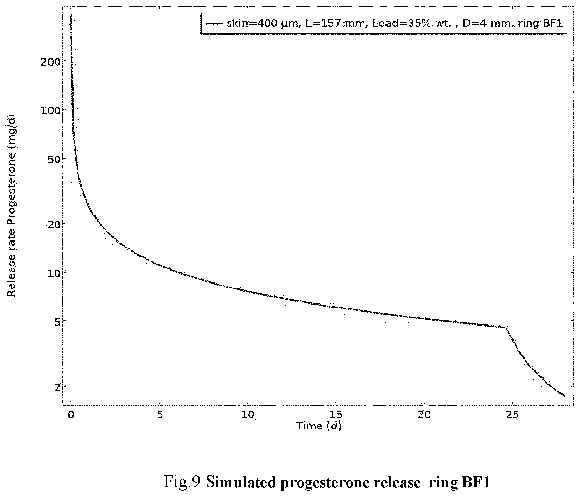

Fig. 9 shows the simulated progesterone release in ring BF1.

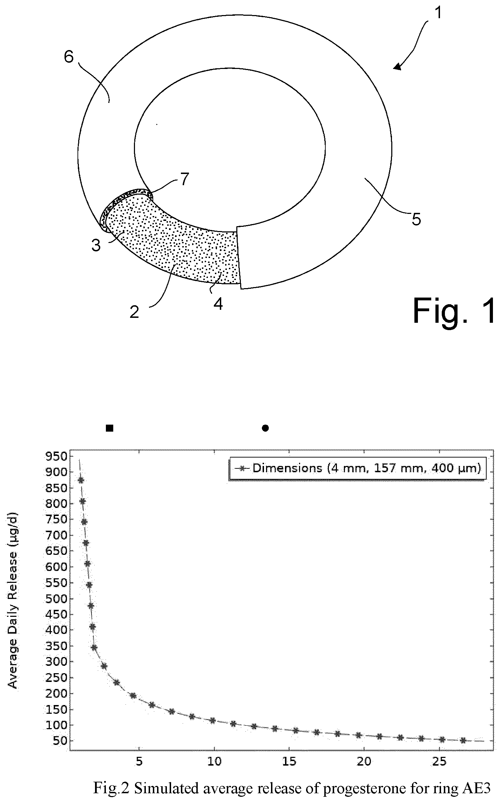

Fig. 1 shows a preferred embodiment of an intravaginal ring (IVR) 1 according to the invention. In said embodiment the IVR comprises a core 2 made of a first ethylene-vinyl acetate copolymer 3 and comprising an estrogenic steroid 4, and a sheath 5 made of a second ethylene-vinyl acetate copolymer 6 and comprising a progestational steroid 7.

### Example 1

In order to evaluate the effect of outer diameter and sheath thickness the release rate of a series of intra-vaginal rings has been evaluated using computational modeling. The model takes into account diffusion of the drug which is driven by the concentration gradient in the system and the slow dissolution of progesterone (P4) crystals dispersed in the sheath and it assumes the receiving medium to be a perfect sink. The cross-sectional diameter of the ring determines the surface area of the systems and hence larger diameter rings are assumed to display proportionally higher drug release.

The thickness of the sheath determines the amount of progesterone present in the ring, assuming progesterone concentration is not varied. The amount of drug substance loaded in the delivery system is an important design parameter as the progesterone release should be sustained over the intended duration of use and on the other hand a large excess should be avoided for economic and environmental reasons.

The evaluated rings are made from EVA28 which is used for both core and sheath. During storage redistribution of dissolved progesterone and estradiol, due to internal diffusion processes, will take place. A fraction of the drug initially loaded in the core (i.e., estradiol (dissolved)) will diffuse into the sheath, and this process will continue until the ring is equilibrated, a process that can take several weeks depending on sheath thickness and storage conditions. The estradiol loading listed in table 1 will result after equilibration in a uniform concentration in the ring, including sheath and core, of 0.27 wt%, which corresponds with the estradiol saturation concentration in EVA28 at 37°C. The thermodynamic equilibrium of the progesterone-loaded sheath (see Table 1) in the ring will result in a progesterone concentration of 30 wt% in the sheath and 1.7 wt% in the core.

The simulations confirm the expectation of the skilled person that in this specific case sheath thickness does not affects estradiol release because the drug is homogeneously distributed after equilibration and no partitioning between core and sheath will take place as both are made of EVA28.

The release rate of a series of nine rings with varying sheath thickness and cross-sectional diameters is given in table 1. From this table it becomes apparent that the release rate of progesterone (P4) and estradiol (E2) scales with cross-sectional diameter.

**Table 1**

| | **Dimensions** | | | **Drug loading** | | **EST release** | | | | **PGN release** | | |
|---|---|---|---|---|---|---|---|---|---|---|---|---|
| Delivery system | Diameter (mm) | Length (mm) | Sheath (µm) | Core wt% E2 | Sheath wt% P4 | Burst (µg/day) | Average daily release (2-28) (µg/day) | Average daily release (1-28) (µg/day) | Day 28 release (µg/day) | Burst (mg/day) | Average daily release (2-28) (mg/day) | Average daily release (1-28) (mg/day) |
| AE1 | 4 | 157 | 400 | 0.43272 | 32.895 | 940.48 | 109.45 | 139.13 | 48.761 | 52.641 | 6.3179 | 7.9723 |
| AE2 | 4 | 157 | 500 | 0.49232 | 32.093 | 940.45 | 109.45 | 139.13 | 48.763 | 50.601 | 6.9361 | 8.4956 |
| AE3 | 4 | 157 | 600 | 0.56514 | 31.564 | 940.41 | 109.45 | 139.12 | 48.759 | 53.497 | 6.9427 | 8.6054 |
| AE4 | 5 | 157 | 400 | 0.39251 | 33.902 | 1183.7 | 146.73 | 183.77 | 72.768 | 64.158 | 8.1184 | 10.12 |
| AE5 | 5 | 157 | 500 | 0.43272 | 32.895 | 1183.6 | 146.73 | 183.76 | 72.769 | 64.344 | 8.8515 | 10.833 |
| AE6 | 5 | 157 | 600 | 0.47945 | 32.226 | 1183.6 | 146.73 | 183.76 | 72.764 | 65.038 | 8.8584 | 10.865 |
| AE7 | 6 | 157 | 400 | 0.36873 | 34.914 | 1426.8 | 183.48 | 227.89 | 95.565 | 77.09 | 9.9179 | 12.317 |
| AE8 | 6 | 157 | 500 | 0.39881 | 33.7 | 1426.8 | 183.48 | 227.88 | 95.564 | 79.327 | 10.763 | 13.212 |
| AE9 | 6 | 157 | 600 | 0.43272 | 32.895 | 1426.7 | 183.47 | 227.88 | 95.56 | 76.649 | 10.771 | 13.124 |

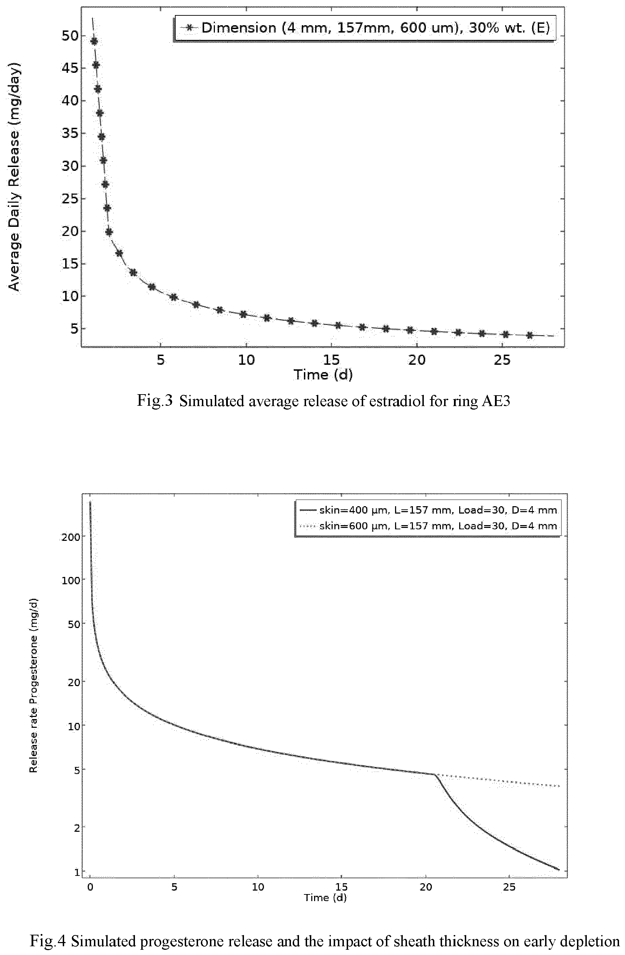

Figure 2 and 3 shows the simulated average release for ring AE3 of progesterone and estradiol respectively. As can be seen from said figures that the release rate of the steroid decreases over the treatment period of 28 days.

Figure 4 displays the simulated release of progesterone from the rings A1 and A3. This simulation illustrates early depletion, which occurs when the sheath thickness is insufficient and contains insufficient progesterone to sustain the release over the intended duration of use of 28 days.

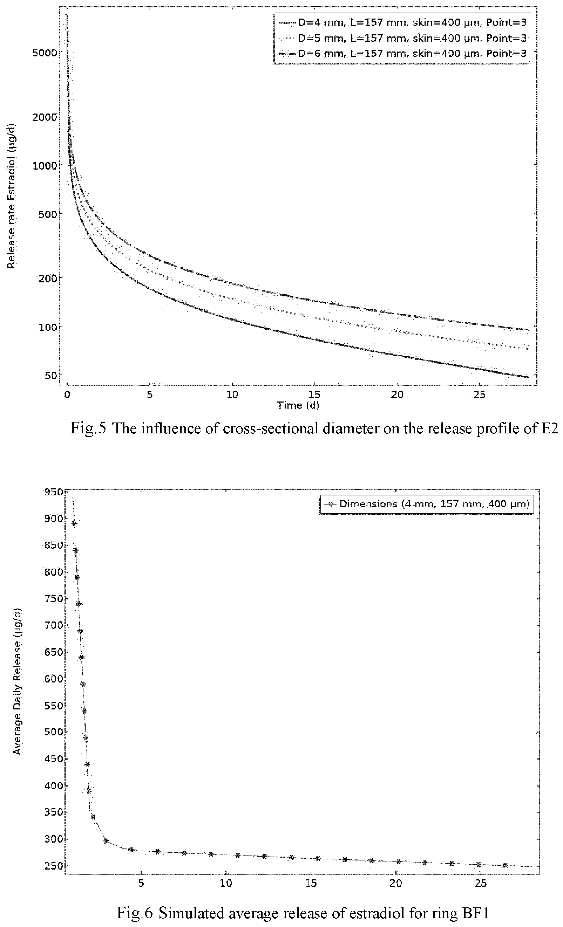

The influence of cross-sectional diameter on the release profile of E2 is demonstrated in figure 5. It should be noted that the release rate visualized in figure 4 and 5 are the actual release rates. Hence the burst appears much higher as the release in the very early moments of eluting is of course much higher than the day one average.

### Example 2

In order to evaluate the effect of the dispersed crystals of Estradiol E2 in the core and the sheath thickness on the release rate, a series of intra-vaginal rings has been evaluated using computational modeling.

The release rate of a series of three rings with varying sheath thickness is given in table 2. From this table, it becomes apparent that the release rate of Estradiol scales with the sheath thickness. Furthermore, the delivery systems (BF1, BF2, and BF3) provide a sustained and high release of Estradiol even at day 28. The change of sheath thickness has no significant effect on the release rate of progesterone.

**Table 2**

| | **Dimensions** | | | **Drug loading** | | **EST release** | | | | **PGN release** | | |
|---|---|---|---|---|---|---|---|---|---|---|---|---|
| Delivery system | Diameter (mm) | Length (mm) | Sheath (µm) | Core wt% E2 | Sheath wt% P4 | Burst (µg/day) | Average daily release (2-28) (µg/day) | Average daily release (1-28) (µg/day) | Day 28 release (µg/day) | Burst (mg/day) | Average daily release (2-28) (mg/day) | Average daily release (1-28) (mg/day) |
| BF1 | 4 | 157 | 400 | 10.153 | 37.866 | 940.59 | 268.3 | 292.31 | 249.27 | 58.163 | 7.4114 | 9.2239 |
| BF2 | 4 | 157 | 500 | 10.211 | 37.073 | 940.41 | 217.51 | 243.33 | 201.14 | 58.13 | 7.6388 | 9.442 |
| BF3 | 4 | 157 | 600 | 10.282 | 36.549 | 940.4 | 183.96 | 210.97 | 166.08 | 58.401 | 7.6437 | 9.4565 |

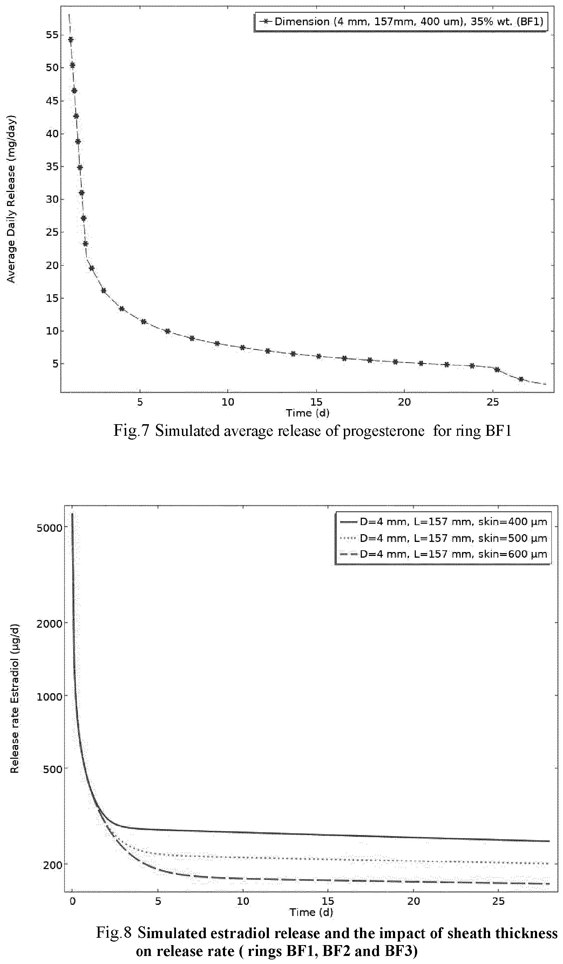

Figure 6 displays the average daily release from ring BF1. The ring provides sufficient estradiol during the whole period of use, 28 days. The increase of the sheath thickness from 400 µm (ring BF1) to 600 µm (ring BF3) reduces the average daily release by almost 30 %, as shown in Table 2 and Figure 8.

Figure 7 displays the simulated release of Progesterone from the ring BF1. Even at a high drug load of 35 % wt, the early depletion occurs, informing about the insufficient Progesterone inside the ring to sustain the release over the intended duration of use of 28 days.

Using the intravaginal ring according to the present the inventors have found that it is possible to attain independent and optimal release of the two active ingredients; an estrogenic steroid and an progestational steroid without the need for complex assembly of parts and without the need to use sophisticated multi-layer extrusion technology.

The intravaginal ring has a simple and inexpensive design, and can therefore be used equally well both privately and in medical or hospital facilities.

Modifications and combinations of the above principles and designs are foreseen within the scope of the present invention.

## Claims

1. An intravaginal ring comprising
- a core (2) comprising a first ethylene-vinyl acetate copolymer (3) having a vinyl acetate content from 26 to 40%, and an estrogenic steroid (4), and
- a sheath (5) comprising a second ethylene-vinyl acetate copolymer (6) having a vinyl acetate content from 20 to 40%, and at least 10 wt% of a progestational steroid (7) based on the weight of the sheath.

2. An intravaginal ring according to claim 1, wherein the sheath comprises at least 15 wt% of the progestational steroid based on the weight of the sheath, preferably at least 20 wt%, and even more preferred at least 30 wt%.

3. An intravaginal ring according to claim 1 or 2, wherein the sheath comprises 40 wt% or less of the progestational steroid based on the weight of the sheath, preferably 35 wt% or less.

4. An intravaginal ring according to any of the preceding claims, wherein the progestational steroid is dispersed and/or incorporated in the second ethylene-vinyl acetate copolymer in the form of particles, such as crystals.

5. An intravaginal ring according to any of preceding claims, wherein the estrogenic steroid is dissolved in the first ethylene-vinyl acetate copolymer at or below the saturation concentration of said estrogenic steroid at 25°C.

6. An intravaginal ring according to any of the claims 1 to 4, wherein the estrogenic steroid is dispersed and/or incorporated in the first ethylene-vinyl acetate copolymer in the form of particles, e.g. crystals.

7. An intravaginal ring according to claim 6, wherein the core comprises at least 10 wt% of the estrogenic steroid based on the weight of the core, preferably at least 15 wt% of, and even more preferred at least 20 wt%.

8. An intravaginal ring according to claim 4 or any of the claims 6 to 8 wherein the particles of the progestational steroid and optionally the estrogenic steroid in the second and optionally the first ethylene-vinyl acetate copolymer, have a particle size of between 3 µm and 40 µm, preferably between 8 µm and 24 µm, and even more preferred between 10 and 24 µm.

9. An intravaginal ring according to any of the preceding claims, wherein the first ethylene-vinyl acetate copolymer has a vinyl acetate content of 26%, 33% or 40%.

10. An intravaginal ring according to any of the preceding claims, wherein the second ethylene-vinyl acetate polymer has a vinyl acetate content of 24%, 28%, 33% or 40%.

11. An intravaginal ring according to any of the preceding claims, wherein the estrogenic steroid is estradiol

12. An intravaginal ring according to any of the preceding claims, wherein the progestational steroid is a selected from the group consisting of progestogen, etonogestrel, levonorgestrel, d-1-norestrel and norethindrone.

13. An intravaginal ring according to any of the preceding claims, wherein the thickness of the sheath is between 0.05 mm and 3 mm, preferably between 0.05 mm and 2 mm, more preferably between 0.1 mm and 2 mm and even more preferably between 0.1 mm and 0.6 mm.

14. An intravaginal ring according to any of the preceding claims, wherein the cross-sectional diameter of the core is from between 2 and 8 mm, more preferably between 3 mm and 6 mm and even more preferably from 4 mm to 5 mm.

15. An intravaginal ring according to any of the preceding claims, wherein the outer diameter of the intravaginal ring ranges from 45 mm to about 65 mm, preferably around 54 mm.

16. A method of manufacturing the intravaginal ring according to any of the claims 1 - 15, said method comprises
a. providing a core comprising a first ethylene-vinyl acetate copolymer having a vinyl acetate content from 26 to 40%, and an estrogenic steroid,
b. providing a sheath of comprising a second ethylene-vinyl acetate copolymer having a vinyl acetate content from 20 to 40%, and at least 10 wt% of an progestational steroid based on the weight of the sheath, and
c. co-extruding the core and sheath into a fiber, and
d. cutting the fiber into an appropriate length thereby providing a fiber element, and
e. combining the ends of fiber element to form the intravaginal ring.

17. A method according to claim 16, wherein said method further comprises a cooling step in which the provided fiber from step c. is cooled to a temperature of about 20°C in order to provide crystals of at least the progestational steroid in sheath.

18. Use of the intravaginal ring for providing a substantially zero-order release rate of the estrogenic steroid during a treatment period of at least 28 days.

19. Use according to claim 18, wherein the average release rate of estradiol is from 80 µg/day to 160 µg/day during a treatment period of at least 28 days.

20. Use according to claim 18 or 19, wherein the average release rate of progestogen is from 5 mg/day to 10 mg/day during a treatment period of at least 28 days.
